# EUROPEAN PATENT SPECIFICATION

(11) **EP 0 647 452 B1**
(45) Date of publication and mention of the grant of the patent: **28.08.2002**
(21) Application number: 94115886.7
(22) Date of filing: 07.10.1994
(51) Int. Cl.: A61L 17/00

(54) **Infection-resistant surgical devices and methods of making them**
Infektionsbeständige chirurgische Gegenstände und Verfahren zu deren Herstellung
Articles chirurgicaux résistant aux infections et procédé pour leur fabrication

(30) Priority: 08.10.1993 US 134146
(43) Date of publication of application: 12.04.1995
(73) Proprietor: United States Surgical Corporation, Norwalk, Connecticut 06856 (US)
(72) Inventor: Gruskin, Elliott A., Killingworth, CT 06419 (US); Lee, Daniel R., Madison, CT 06443 (US); Brown, Lloyd S., Guilford, CT 06437 (US)
(74) Representative: HOFFMANN - EITLE

(56) References cited:
- EP-A- 0 207 624
- EP-A- 0 328 421
- EP-A- 0 449 431
- EP-A- 0 494 369
- US-A- 5 037 429
- The Merck Index, 12th Edition, nos. 8647-8678

## Description

The present invention relates to black, infection-resistant surgical devices and to a method for preparing such devices.

Surgical devices for use externally or internally with humans or animals can serve to introduce bacterial, viral, fungal or other undesirable infections. To prevent such contamination, surgical devices can be treated with an antimicrobial agent. For example, U.S. Patent No. 5,019,096 to Fox, Jr. et al. describes applying a coating to a medical device, the coating containing a matrix polymer and antimicrobial agents, preferably a combination of a silver salt and chlorhexidine. EP-A2-328421 discloses using a combination of a silver salt and chlorhexidine in the preparation of medical devices, to improve their resistance to infection. Other examples of antimicrobial devices include U.S. Patent Nos. 3,674,901; 3,705,938; 3,987,797; 4,024,871; and 4,612,337.

Many surgical devices are made from one or more filaments. The filaments can be woven, braided, knitted or combined to provide a non-woven filamentary structure. The filaments used to form surgical devices can be made from natural or synthetic materials which may be absorbable or non-absorbable. Where filaments of a synthetic absorbable polymer are used to form the surgical device, steps must be taken to ensure stability of the polymer if the device is to be stored in a package for extended periods. Methods for improving the storage stability of polymeric articles subject to hydrolytic degradation are described, for example, in U.S. Patent Nos. 5,037,429 and 5,051,272. US-A-5037429 discloses using glycerol to improve the storage stability of surgical devices.

Surgical devices can be dyed to impart a desired color to the device. Frequently, the dye is incorporated directly into the polymer from which the surgical device is manufactured. It is sometimes desirable to employ a black colored surgical device in surgical procedures. Currently, logwood extract and carbon black are believed to be the only materials employed to manufacture black colored surgical devices.

### Summary of the Invention

The present invention is defined in claim 1 hereinbelow. Dependent claims define optional or preferred features.

In a particularly useful embodiment, the antimicrobial agent employed is silver lactate.

Thus, according to the invention, a black-colored surgical suture can be prepared by providing a substrate having interstices or pores contacting the substrate with a composition comprising glycerine and a silver salt, and contacting the impregnated substrate with ethylene oxide. In particularly useful embodiments, the substrate is made from one or more filaments. The process of this invention provides a combination of desired characteristics in the surgical device in an economical manner heretofore unachieved. Specifically, surgical sutures prepared in accordance with the present invention have antimicrobial properties, are black in color, are sterilized and, when the suture includes bioabsorbable materials, exhibits excellent storage stability.

### Detailed Description of Preferred Embodiments

The surgical devices of this invention include a substrate prepared at least in part from one or more filaments. The substrate is contacted with a composition which contains glycerine and an antimicrobial agent.

In particularly useful embodiments, the substrate is made from one or more filaments. The filaments from which the substrate is made are synthetic fiber-forming material. Thus, for example, the filaments can be made from materials such as polypropylene, nylon, polyesters including polyethyleneterephthalate and polybutyleneterephthalate, or synthetic absorbable polymers such as those made from glycolide, lactide, p-dioxanone, trimethylene carbonate and E-caprolactone. Random, block or graft copolymers and blends of the above-mentioned synthetic absorbable materials are also suitable for preparing the filaments.

The substrate can be made from the filaments using any known technique such as for example, braiding, weaving or knitting. The filaments may also be combined to produce a non-woven substrate. The filaments themselves may be drawn, oriented, crinkled, twisted or commingled or air entangled to form yarns as part of the substrate forming process. The substrate should include interstices or spaces between the filaments (or overlapping areas of the same filament) into which the composition with which the substrate is contacted can flow. It is also envisioned that the substrate may be a porous sponge-like product impregnated by the application of a suitable composition under pressure.

Once formed the substrate is contacted with a composition which contains glycerine and an antimicrobial agent. The anti-microbial agent comprises a silver salt. Suitable silver salts include silver acetate, silver benzoate, silver carbonate, silver iodate, silver iodide, silver lactate, silver laurate, silver nitrate, silver oxide, silver palmitate, silver protein, silver sulfadiazine or combinations thereof. A particularly useful silver salt is silver lactate.

The composition contains from about 0.001 to about 15 percent of the antimicrobial agent by weight. The exact amount of the antimicrobial agent will depend on a number of factors such as the particular agent employed, the configuration of the substrate and the presence or absence of other components in the composition. Preferably, the composition contains from about 5 to about 7 percent silver salt by weight. In a particularly useful embodiment, the composition contains from about 6.0 to about 6.5 percent silver lactate.

If necessary or desirable, the glycerine and antimicrobial agent can be dissolved in any suitable solvent or combination of solvents prior to use. To be suitable, the solvent must (1) be miscible with the glycerine/antimicrobial agent components at the concentration of the latter, (2) have a sufficiently high vapor pressure to be readily removed by evaporation, (3) not appreciably affect the integrity of the polymeric article and (4) capable, in combination with the glycerine and antimicrobial agent of wetting the surface of the substrate. Applying these criteria to a preferred composition, glycerine and silver lactate, water is a suitable solvent carrier.

The composition with which the substrate is contacted will hereinafter be referred to as the "impregnating agent".

Preparing the impregnating agent is a relatively simple procedure. For example, in the case of glycerine and silver lactate, the desired amount of glycerine is first introduced to a container, followed by the addition thereto of the desired amount of silver lactate. Water as solvent is added to the mixture of glycerine and silver lactate and the solution is then thoroughly mixed to combine the compounds.

Generally, the impregnating agent is comprised of a mixture of a silver salt, such as silver lactate, and a water soluble hygroscopic polyhydroxy compound, such as glycerine, in a weight ratio of between about 1:1 to about 1:10, most preferably 1:7, respectively. When water is utilized in the preparation of the impregnating agent, the solvent is employed in amounts to provide a solution concentration of from about 20% to about 50%, preferably about 30% to about 45%, by weight of the compound of the polyhydroxy compound, such as glycerine, based on the total weight of the solution.

Upon contacting the substrate with the impregnating agent, the impregnating agent flows into the interstices between the filaments from which the substrate is formed.

Any known technique may be employed for contacting the substrate with the impregnating agent. Suitable techniques include dipping, spraying, wiping and brushing. Where the substrate is in the form of a braided suture, techniques used for applying a coating or other treatment to a fiber may be employed to contact the substrate with the composition. The impregnating agent may be applied to a yarn which is then woven or otherwise processed to form the surgical suture. Preferably, however, the impregnating agent is applied to the substrate in its final form.

The amount of the impregnating agent applied to the substrate should be an effective amount to provide antimicrobial properties. The exact amount will depend upon, inter alia, the configuration of the substrate and the formulation of the composition. Typically, the impregnating agent will be applied in an amount from about 2 to about 25 weight percent (excluding any solvent) by weight of the substrate. Preferably the impregnating agent is applied in an amount from about 5 to about 15 weight percent (excluding any solvent) by weight of the substrate.

If the substrate has been previously coated with a substance through which the impregnating agent cannot pass, the substrate should be treated to crack such coating prior to contact with the impregnating agent. This will allow the impregnating agent to flow into the interstices of the substrate. Any known technique may be used to crack the impenetrable coating such as for example calendaring. Alternatively, the substrate should be impregnated with the glycerine composition prior to application of the coating.

If a solvent is used in the impregnating composition, a drying step may be employed to flash off the solvent.

Once the substrate is contacted with the impregnating agent, the surgical suture is sterilized by exposure to gaseous ethylene oxide. In accordance with the present invention, a surgical suture having a black color is provided by sterilization with ethylene oxide.

The present invention is useful in conjunction with other known and conventional packaging techniques and materials. As previously stated, another advantage of the present invention lies in its ability to provide enhanced storage stability in a polymeric article susceptible to hydrolytic degradation without having to eliminate all but a small amount of moisture from the article and maintain the article in an especially dry environment until the final package sealing operation as disclosed in U.S. Patent Nos. 3,728,839 and 4,135,622. While the present invention can be practiced with a suture or other article which has been treated in this manner, there is no necessity of doing so and for reasons of simplicity, economy and production efficiency, it is preferred that the article to be contacted with impregnating agent in accordance with this invention does not receive the treatment described in the aforesaid patents. Preferably, the packaging and moisture equilibration techniques employed are those described in U.S. Patent No. 5,051,272.

It can be advantageous to employ the impregnating agent as a carrier for one or more medico-surgically useful substances, e.g., those which accelerate or otherwise beneficially modify the healing process when applied to a wound or surgical site. In general, any biologically active material which is soluble in and otherwise compatible with the selected impregnating agent can be incorporated therein in therapeutically useful amounts. So, for example, a suture can be filled with an impregnating agent containing a therapeutic agent which will be deposited at the suture site. The therapeutic agent may be chosen for its capability for promoting wound repair and/or tissue growth or for specific indications such as thrombolysis. Antimicrobial agents such as broad spectrum antibiotics (gentamicin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or several growth promoting factors can be added to the impregnating agent, e.g., fibroblast growth factor, platelet derived growth factor, macrophage derived growth factor, alveolar derived growth factor, monocyte derived growth factor, magainin, and so forth. Some therapeutic indications are: glycerine with tissue or kidney plasminogen activator to cause thrombosis, superoxide dismutase to scavenge tissue damaging free radicals, tumor necrosis factor for cancer therapy or colony stimulating factor and interferon, interleukin-2 or other lymphokine to enhance the immune system.

The following Example is illustrative of surgical suture to which a black colour can be imparted by sterilization with ethylene oxide, in accordance with this invention.

### EXAMPLE

A 4/0 size braid of 90/10 glycolide/lactide copolymer filaments is prepared. The braid preferably has the structure described in U.S. Patent No. 5,059,213.

The braid is then contacted with an impregnating agent having the composition shown in Table A. An identical braid is contacted with a similar composition containing no silver salt to serve as a control. The composition employed to prepare the control is also given in Table A.

**TABLE A**

| | Example | Control |
|---|---|---|
| glycerine | (65g) 40.2% | 40.2% |
| silver lactate | (10g) 6.2% | -- |
| calcium lactate | -- | (10g) 6.2% |
| water | (86g) 53.5% | (86g) 53.5% |
| E.coli | <1.81±0.25 | 4.66±0.66 |
| S.aureus | 5.88±1.2 | 6.00±1.2 |

The amount of impregnating agent on the braid was 10%. A needle is then attached to the braid.

Sub-dermal implant sites on mice were contaminated with 10^{4.69} S. aureus or 10^{4.34} E. coli and then received an implant of size 4/0 suture made either in accordance with this invention or a control suture. The number of bacteria at each suture tract was quantitated after 4 days. The results are reported in Table A and reflect the average and standard deviation for 12 readings.

The sutures made in accordance with the present invention were very effective in sterilizing 75% (9/12) of the implant sites contaminated with 10^{4.34} E. coli.

The claims which follow identify embodiments of the invention additional to those described in detail above.

## Claims

1. Use of an impregnating agent to simultaneously impart anti-microbial properties and a black colour to a synthetic surgical suture which includes a substrate having interstices or pores, by
(a) impregnating the surgical suture with the impregnating agent, and
(b) contacting the impregnated surgical suture with ethylene oxide,
the agent essentially being composed of a silver salt, a soluble hygroscopic polyhydroxy compound, and a solvent.

2. Use of an impregnating agent as claimed in claim 1, wherein the silver salt is silver lactate, the soluble hygroscopic polyhydroxy compound is glycerine, and/or the solvent is water.

3. Use of an impregnating agent as claimed in claim 1 or 2, wherein the weight ratio of the silver salt and the soluble hydroscopic polyhydroxy compound ranges between about 1:1 and about 1:10, and is preferably 1:7.

4. Use of an impregnating agent as claimed in claim 2 or 3, with water as the solvent, wherein the solvent is employed in amounts to provide a solution concentration of from about 20% to about 50%, preferably from about 30% to about 45%, by weight of the compound of the soluble hygroscopic polyhydroxy compound and based on the total weight of the solution.

5. Use of an impregnating agent as claimed in any one of the preceding claims, wherein the surgical suture is a braided yarn.

6. Use of an impregnating agent as claimed in any one of the preceding claims, wherein in step (a) said surgical suture is impregnated with from about 0.005 to 0.100 grams of said impregnating agent per gram of said surgical suture.

7. Use of an impregnating agent as claimed in any one of the preceding claims, wherein said surgical suture is formed at least in part of a bio-absorbable material.

## Patentansprüche

1. Verwendung eines Imprägniermittels, um einem synthetischen chirurgischen Nahtmaterial, das ein Substrat mit Zwischenräumen oder Poren aufweist, gleichzeitig antimikrobielle Eigenschaften und eine schwarze Farbe zu verleihen, indem
(a) das chirurgische Nahtmaterial mit dem Imprägniermittel imprägniert wird, und
(b) das imprägnierte chirurgische Nahtmaterial mit Ethylenoxid in Kontakt gebracht wird,
wobei das Imprägniermittel im wesentlichen aus Silbersalz, einer löslichen hygroskopischen Polyhydroxy-Komponente, und einem Lösungsmittel besteht.

2. Verwendung eines Imprägniermittels gemäß Anspruch 1, wobei das Silbersalz Silberlactat ist, die lösliche hygroskopische Polyhydroxy-Komponente Glycerin ist, und /oder das Lösungsmittel Wasser ist.

3. Verwendung eines Imprägniermittels gemäß Anspruch 1 oder 2, wobei das Gewichtsverhältnis des Silbersalzes und der löslichen hygroskopischen Polyhydroxy-Komponente sich im Bereich zwischen etwa 1:1 und etwa 1:10 bewegt, und vorzugsweise 1:7 ist.

4. Verwendung eines Imprägniermittels gemäß Anspruch 2 oder 3, mit Wasser als dem Lösungsmittel, wobei das Lösungsmittel in einer Menge verwendet wird, um eine Lösungskonzentration von etwa 20 bis etwa 50 Gew-%, vorzugsweise von etwa 30 bis etwa 45 Gew-% der Komponente der löslichen hygroskopischen Polyhydroxy-Komponente und basierend auf dem Gesamtgewicht der Lösung vorzusehen.

5. Verwendung eines Imprägniermittels gemäß einem der vorhergehenden Ansprüche, wobei das chirurgische Nahtmaterial ein geflochtener Garn ist.

6. Verwendung eines Imprägniermittels gemäß einem der vorhergehenden Ansprüche, wobei im Schritt (a) das chirurgische Nahtmaterial mit von etwa 0,005 bis etwa 0,100 Gramm des Imprägniermittels per Gramm des chirurgischen Nahtmaterials imprägniert wird.

7. Verwendung eines Imprägniermittels gemäß einem der vorhergehenden Ansprüche, wobei das chirurgische Nahtmaterial zumindest teilweise aus einem bioabsorbierbaren Material gebildet ist.

## Revendications

1. Utilisation d'un agent d'imprégnation pour donner des propriétés anti-microbiennes et une couleur noire à une suture chirurgicale synthétique qui comprend un substrat présentant des interstices ou des pores, par les étapes consistant à :
(a) imprégner la suture chirurgicale avec l'agent d'imprégnation, et
(b) mettre en contact avec un oxyde d'éthylène la suture chirurgicale ainsi imprégnée,
l'agent étant essentiellement composé d'un sel d'argent, d'un composé polyhydroxy hygroscopique soluble, et d'un solvant.

2. Utilisation d'un agent d'imprégnation telle que revendiquée à la revendication 1, dans laquelle le sel d'argent est le lactate d'argent, le composé polyhydroxy hygroscopique soluble est la glycérine, et/ou le solvant est l'eau.

3. Utilisation d'un agent d'imprégnation telle que revendiqué à la revendication 1 ou 2, dans laquelle le rapport pondéral du sel d'argent et du composé polyhydroxy hygroscopique soluble est dans une gamme comprise entre environ 1:1 et environ 1:10, et est de préférence de 1:7.

4. Utilisation d'un agent d'imprégnation telle que revendiquée à la revendication 2 ou 3, avec l'eau en tant que solvant, dans laquelle le solvant est utilisé dans des quantités permettant de créer une concentration de la solution d'environ 20% à environ 50%, de préférence d'environ 30% environ 45%, par poids du composé polyhydroxy hygroscopique soluble et sur la base du poids total de la solution.

5. Utilisation d'un agent d'imprégnation telle que revendiquée dans une quelconque des revendications précédentes, dans laquelle la suture chirurgicale est un fil tressé.

6. Utilisation d'un agent d'imprégnation telle que revendiquée dans une quelconque des revendications précédentes, dans laquelle, dans l'étape (a), ladite suture chirurgicale est imprégnée avec environ 0,005 à 0,100 gramme dudit agent d'imprégnation par gramme de ladite suture chirurgicale.

7. Utilisation d'un agent d'imprégnation telle que revendiquée dans une quelconque des revendications précédentes, dans laquelle ladite suture chirurgicale est formée au moins en partie d'une matière bio-absorbable.
